(19) Europäisches Patentamt
European Patent Office
Office européen des brevets

(11) **EP 3 859 315 B1**

(12) **EUROPEAN PATENT SPECIFICATION**

(45) Date of publication and mention
of the grant of the patent:
**28.02.2024 Bulletin 2024/09**

(21) Application number: **19933578.7**

(22) Date of filing: **16.10.2019**

(51) International Patent Classification (IPC):
**G01N 21/65** (2006.01)    **G01N 33/02** (2006.01)

(52) Cooperative Patent Classification (CPC):
**G01N 21/65; G01N 33/025;** G01J 2003/4424;
G01N 2021/8466; G01N 2201/129

(86) International application number:
**PCT/CN2019/111533**

(87) International publication number:
**WO 2020/252999 (24.12.2020 Gazette 2020/52)**

(54) **METHOD FOR TESTING WATER CONTENT AND WATER DISTRIBUTION OF CELLULAR LEVELS IN FRUIT AND VEGETABLE TISSUES ON BASIS OF RAMAN SPECTRUM**

VERFAHREN ZUM TESTEN DES WASSERGEHALTS UND DER WASSERVERTEILUNG AUF ZELLULÄRER EBENE IN OBST- UND GEMÜSEGEWEBEN AUF RAMAN-SPEKTRUMBASIS

PROCÉDÉ DE TEST DE TENEUR EN EAU ET DE DISTRIBUTION D'EAU DE NIVEAUX CELLULAIRES DANS DES TISSUS DE FRUITS ET LÉGUMES SUR LA BASE D'UN SPECTRE RAMAN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2019   CN 201910526973**

(43) Date of publication of application:
**04.08.2021   Bulletin 2021/31**

(73) Proprietor: **South China University of Technology Guangzhou, Guangdong 510640 (CN)**

(72) Inventors:
• **SUN, Da-Wen**
  **Guangzhou, Guangdong 510640 (CN)**
• **LI, Dongmei**
  **Guangzhou, Guangdong 510640 (CN)**
• **ZHU, Zhiwei**
  **Guangzhou, Guangdong 510640 (CN)**

(74) Representative: **Schulz Junghans Patentanwälte PartGmbB Großbeerenstraße 71 10963 Berlin (DE)**

(56) References cited:
**EP-A1- 3 164 046      CN-A- 106 290 292**

CN-A- 107 209 934    CN-A- 110 208 244

• PU YUAN-YUAN ET AL: "Vis-NIR hyperspectral imaging in visualizing moisture distribution of mango slices during microwave-vacuum dr", FOOD CHEMISTRY, ELSEVIER LTD, NL, vol. 188, 27 April 2015 (2015-04-27), pages 271-278, XP029210671, ISSN: 0308-8146, DOI: 10.1016/J.FOODCHEM.2015.04.120
• RAOUL VYUMVUHORE ET AL: "Effects of atmospheric relative humidity on Stratum Corneum structure at the molecular level: ex vivo Raman spectroscopy analysis", ANALYST, vol. 138, no. 14, 1 January 2013 (2013-01-01), page 4103, XP055766760, UK ISSN: 0003-2654, DOI: 10.1039/c3an00716b
• PU, Yuanyuan et al. ,: "Vis-NIR hyperspectral imaging in visualizing moisture distribution of mango slices during microwave-vacuum drying,", Food Chemistry,, 27 April 2015 (2015-04-27), XP029210671, ISSN: 0308-8146, DOI: 20200302123032Y
• VYUMVUHORE, Raoul et al. ,: "Effects of atmospheric relative humidity on Stratum Corneum structure at the molecular level: ex vivo Raman spectroscopy analysis,", Analyst,, 31 December 2013 (2013-12-31), XP055766760, ISSN: 0003-2654, DOI: 20200302123350Y

**Description**

**FIELD OF THE INVENTION**

The present invention belongs to the technical field of spectral detection, and specifically relates to a method for testing the cellular level water content and distribution in fruit and vegetable tissues based on Raman spectroscopy.

**BACKGROUND OF THE INVENTION**

As one of the indispensable foods in people's daily life, fruits and vegetables have high water content. However, due to the heterogeneity, porosity and hygroscopicity of their tissue structure, the distribution of water in the tissues is very complex. Based on the research of biohistology, we usually think that the water in fruits and vegetables is mostly distributed in vacuole, cytoplasm, cell wall and extracellular space. According to the bonding strength of water with other substances in tissues, water in fruits and vegetables was divided into free water, immobilized water and bound water, and the water in the vacuole and extracellular space was defined as free water, the water in the cytoplasm was defined as immobilized water, and the water in the cell wall was defined as bound water. At present, in order to accurately determine the content and distribution of water in different bonding states in fruits and vegetables, some methods has been employed, including Marlin Chick experiment, differential scanning calorimetry, differential thermal analysis, bioimpedance analysis, and low-field nuclear magnetic resonance (NMR) response. Each of these methods has its own advantages and disadvantages. The contents of bound water and free water in plant tissues can be determined by the Malin Chick experiment, differential scanning calorimetry, and differential thermal analysis; however, these methods can only divide the measured water into two kinds, i.e., bound water and free water, and can neither distinguish the immobilized water between the two in more detail, nor distinguish whether the water is distributed inside or outside the cell. The bioimpedance analysis method can determine the content of intracellular water and extracellular water, but it cannot evaluate the bonding strength of the intracellular water and extracellular water. The low-field NMR method is currently the most convenient and quick method for determining free water, immobilized water, and bound water. It can be used to determine the bonding strength of water with other substances according to the relaxation time $T_2$ of water in the sample, and finally divide the water in the sample into free water (with the longest relaxation time), immobilized water and bound water (with the shortest relaxation time), and determine the content ratio of water in different states in the sample according to the area ratio of the three waters. However, this method cannot provide information on the distribution and location of water with different bonding states, and it is difficult to quantitatively analyze the content of water in the sample.
Pu Yuan-Yuan et al. in "Vis-NIR hyperspectral imaging in visualizing moisture distribution of mango slices during microwave-vacuum dr", Food chemistry, Elsevier, vol. 188, 27 April 2015 (2015-04-27), pages 271-278, applies hyperspectral imaging and waveband selection strategies for non-destructively and rapidly measuring and visualizing moisture content during drying process of mango slices.
Raoul Vyumvuhore et al. in "Effects of atmospheric relative humidity on Stratum Corneum structure at the molecular level: ex vivo Raman spectroscopy analysis", Analyst, vol. 138, no. 14, 1 January 2013 (2013-01-01), page 4103 teaches an estimation for content types of water, bound and free, on human Stratum Corneum using Raman spectroscopy and curve fitting of spectral bands in the spectral region in the range of 3.100 cm$^{-1}$ and 3.700 cm$^{-1}$.
EP 3 164 046 A1 discloses a method for characterizing a type or nature of a sample or tissue by analyzing spectral sub intervals of a Raman spectrum in two distinct wavelength ranges.
Despite these findings, there is currently no method to directly determine the cellular level water content and distribution in fruit and vegetable tissues.

**CONTENTS OF THE INVENTION**

In order to overcome the shortcomings and deficiencies of the prior art, an object of the present invention is to provide a method for testing the cellular level water content and distribution in fruit and vegetable tissues based on Raman spectroscopy.
This method can visually image and quantitatively analyze the distribution and content of water in fruit and vegetable tissues at the cell level, so as to accurately obtain the information on the location and content of water with different bonding states (i.e., free water, immobilized water, and bound water) in fruit and vegetable tissues.
The object of the present invention is achieved through the following technical solution:
A method for testing cellular level water content and distribution in fruit and vegetable tissues based on Raman spectroscopy is provided, comprising the following steps:

(1) cutting fruits and vegetables to be tested into a sample;

(2) placing the sample on the stage of a laser confocal microscope for imaging spectrum acquisition; specifically, selecting a cell region from the sample by the objective lens of the laser confocal microscope, then dividing the selected cell region into a grid to obtain uniformly distributed intersection points, then marking the corresponding coordinate information of each intersection point in the selected cell region, and then scanning each intersection point to obtain the corresponding Raman spectrum of water at each intersection point in the cell region;
wherein the step size of the grid is 3-5 $\mu$m;

(3) smoothing and removing the fluorescence background of the Raman spectra obtained in step (2), and then performing Gaussian peak fitting; wherein five sub-peaks at 3000-3800 cm$^{-1}$ and two or three sub-peaks at 2700-3000 cm$^{-1}$ are obtained for each Raman spectrum;

(4) summing up the areas of the five sub-peaks at 3000-3800 cm$^{-1}$ of each Raman spectrum to obtain the corresponding water content A at the intersection point in the cell region, and then determining the bonding state of the corresponding water molecules at the intersection point according to R, the area ratio of the sub-peak centered at 3410-3440 cm$^{-1}$ to the sub-peak centered at 3200-3220 cm$^{-1}$; and

(5) by combining the coordinate information of each intersection point, using the corresponding water content A and ratio R at all the intersection points as pixels for pseudocolor imaging to obtain the distribution of water content and bonding states at the cellular level in the fruit and vegetable tissues.

The fruits and vegetables in step (1) are preferably one of apples, potatoes, grapes, pears, and cabbage stems.

The sample in step (1) is peeled fruit and vegetable, the shape of which is preferably circular, and the size of which is determined by the instrument used to store or test the sample, preferably is 12 mm × 2 mm (diameter × thickness).

Before and during the test, the sample in step (1) is preferably stored in a quartz chamber, which sealed with a quartz cover glass of 0.3 mm in thickness. The temperature of the chamber is kept at 2°C to 10°C and the humidity in the chamber is kept greater than 80%. The chamber can effectively inhibit the water evaporation of the sample in the environment during the test, so as to maintain the stability of the water content of the sample during the test.

The size of the cell region in step (2) is determined by the size of the cells, and the average diameter of the conventional fruit and vegetable cells is 100-300 $\mu$m.

The imaging spectrum acquisition in step (2) is preferably carried out at a depth of 50-100 $\mu$m, which can minimize the influence of the slicing process on the water content in the tissue cells, and allow going deep into the cells to measure the water distribution in the cells.

The laser used for the imaging spectrum acquisition in step (2) is preferably a 532 nm laser, the grating spectrometer is set as 600 gr/mm, the confocal hole is 500 $\mu$m, and the scanning range is 2700-3800 cm$^{-1}$; the acquisition conditions are preferably as follows: the acquisition time is 3-5 s, the accumulation times are 2-3 times, and the laser energy attenuation is 25% to 50%.

The magnification of the objective lens in step (2) is preferably 10×.

In step (3), preferably the spectrums are smoothed by the Savitzky-golay algorithm in the Matlab software; and adaptive iteratively reweighted penalized least squares background subtraction algorithm (airPLS algorithm) is preferably applied to substract the baseline of the spectra.

The "Gaussian peak fitting" in step (3) is carried out preferably by using the Matlab software with the Peakfit function.

The Peakfit function, as a nonlinear iterative curve fitting function with the Gaussian equation as the peak shape, can obtain the maximum fitting determination coefficient by adjusting the number of iterations, peak shape, and peak position.

The "Gaussian peak fitting" in step (3) is preferably a fixed-peak-position Gaussian peak fitting, that is, the fixed-peak-position Gaussian iterative curve fitting algorithm. The Gaussian curve fitting is performed on the spectrum with fixed peak position. The method for determining the peak position is as follows: randomly selecting fifty Raman spectra, and using the Peakfit software to perform iterative peak fitting to divide each Raman spectrum into seven or eight sub-peaks; and then averaging the peak position information of each sub-peaks obtained from the fifty Raman spectra to get the average peak position information of the seven or eight sub-peaks, which can be used as the peak position of the fixed-peak-position Gaussian peak fitting.

The Peakfit software is more preferably the software Peakfit v4.12.

In step (3), the two or three sub-peaks at 2700-3000 cm$^{-1}$ are CH stretching vibration bands of carbohydrates; the five sub-peaks at 3000-3800 cm$^{-1}$ are the stretching vibration bands of O-H of water molecules which effected by the hydrogen bonds, and they respectively correspond to water molecules with different hydrogen bonding state.

The "Gaussian peak fitting" in step (3) can also obtain the determination coefficient of the peak fitting and the relative error between the fitted spectrum and the raw spectrum.

In step (4), the sum of the peak areas of the five sub-peaks at 3000-3800 cm$^{-1}$ represents the content of corresponding water molecules at the intersection point in step (2), i.e., the water content, so it can characterize the water content at this intersection point; the area ratio R of the sub-peak centered at 3410-3440 cm$^{-1}$ to the peak area of the sub-peak centered at 3200-3220 cm$^{-1}$ can reflect the hydrogen bonding state of water molecules, and further reflect the bonding strength of water molecules; wherein the smaller the ratio R is, the higher the bonding strength of water molecules is; and the greater the ratio R is, the higher the degree of freedom of water molecules is.

The "pseudocolor imaging" in step (5) is preferably carried out by using the Matlab software with the Pcolor and Colormap functions with Shading interp used for shading, the pseudocolor map with the water content A as the pixel realizes the visualization of the water content at the cell level, and the pseudocolor map with the ratio R as the pixel realizes the visualization of the water bonding state at the cell level.

In the present invention, the high resolution of the Raman spectroscopy can be used to capture subtle changes in intermolecular or intramolecular hydrogen bonds; the subtle changes in hydrogen bonds are easily affected by the external environment, such as temperature, pressure, ion concentration and space size, so they can be used to study the structure of water molecules. The Raman spectrum of water molecules is a broad peak with two obvious maxima centered around 3240 cm$^{-1}$ and 3440 cm$^{-1}$, respectively. The relative intensity of the two maxima is closely related to the changes in hydrogen bonds; when the hydrogen bonding strength of water molecules is enhanced, the relative intensity of the maximum centered around 3240 cm$^{-1}$ increases; when the hydrogen bonding strength of water molecules is weakened, the relative intensity of the maximum centered around 3440 cm$^{-1}$ increases. For the formation of hydrogen bonds, water molecules can act as electron donors (D) or electron acceptors (A). In order to better study the influence of hydrogen bonds on the Raman spectra of water molecules, the Raman spectra of water at 293 K and 0.1 MPa can be divided by the Gaussian peak fitting algorithm into five sub-peaks centered around 3041 cm$^{-1}$, 3220 cm$^{-1}$, 3430 cm$^{-1}$, 3572 cm$^{-1}$ and 3636 cm$^{-1}$, respectively. According to the studies, these five sub-peaks are sequentially defined as DAA (one electron donor, two electron acceptors), DDAA (two electron donors, two electron acceptors), DA (one electron donor, one electron acceptor), DDA (two electron donors, one electron acceptor), and free-OH without any hydrogen bonding. In these studies, it is concluded that the ratio of the peak area of the DA sub-peak to the peak area of the DDAA sub-peak can be used to characterize the strength of hydrogen bonding around water molecules; the smaller the ratio R of DA/DDAA is, the more water molecules form more hydrogen bonds with surrounding molecules, so that the water molecules have the higher bonding strength; in the opposite case, the water molecules have the higher degree of freedom. Therefore, the ratio R of DA/DDAA can be used to characterize the bonding state of water, so as to determine whether the water is free water or bound water.

Water in fruits and vegetables mainly exists among and within cells, and interacts with other substances (including metal ions, small biological molecules and biological macromolecules) in the cells to form hydrogen bonds, whose strength also affects the state of water existed in cells. With the high resolution of a confocal Raman microscope, the changes in the hydrogen bonds of cellular water can be captured, thereby the state of water in cells can be determined according to the strength of the hydrogen bond, and the content of water molecules can be determined according to the intensity of water molecule signals.

Compared with the prior art, the present invention has the following advantages and beneficial effects:

(1) By combining the confocal Raman microscope imaging technology with mathematical calculation, the present invention provides a method for testing the content of water with different bonding states in fruit and vegetable tissues, and realizes the visualization of water content and water state at the cell level for the first time; therefore, the present invention allows to more intuitively understand the state and location of water existing in the tissue cells, thus providing a basis method for studying the water migration during the fruit and vegetable processing and the influence of the water migration on the tissue structure.

(2) By using the airPLS algorithm, the baseline of the Raman spectrum can be fitted accurately, in addition, it also have great advantages in computing speed, which can process great amount of spectra quickly so that can save the time for spectral preprocessing.

**BRIEF DESCRIPTION OF THE DRAWINGS**

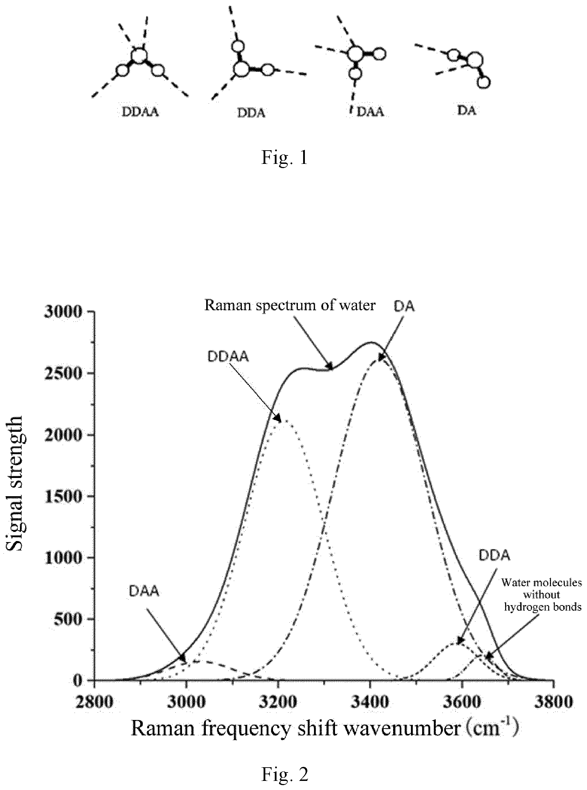

Fig. 1 shows the hydrogen bonding modes of water molecules.

Fig. 2 shows a Raman spectrum and the peak fitting result of water molecules with different hydrogen bonding modes at 293 K and 0.1 MPa.

Fig. 3 is an optical microscopy image of apple tissue at a depth of 50 $\mu$m in Example 1 of the present invention under a 10× objective lens.

Fig. 4 is an optical microscope image of a single cell in a test region at a depth of 50 $\mu$m of the apple tissues tested in Example 1 of the present invention (an enlarged view of the central test region in Fig. 3).

Fig. 5 shows all the raw Raman spectra of each intersection point in the cell region of the apple tested in Example 1 of the present invention after being scanned.

Fig. 6 is the raw, smoothed and baseline-corrected Raman spectrum of a certain original spectrum obtained in Example 1 of the present invention (apple tissue).

Fig. 7 shows the sub-peaks and total peak of a certain preprocessed spectrum obtained by the FPGICF algorithm in Example 1 of the present invention (apple tissue).

Fig. 8 shows the distribution of the determination coefficients (number of spectra) of the preprocessed spectrum for the Gaussian peak fitting in Example 1 of the present invention (apple tissue).

Fig. 9 is a histogram of the distribution of the determination coefficient of the preprocessed spectrum for the Gaussian peak fitting in Example 1 of the present invention (apple tissue).

Fig. 10 shows the cellular level water content distribution of the apple tissue in Example 1 of the present invention.

Fig. 11 shows the cellular level location distribution of water with different bonding state of the apple tissue in Example 1 of the present invention.

Fig. 12 shows the inversion results of the apple tissue obtained by the NMR method.

## DETAILED DESCRIPTION OF THE EMBODIMENTS

The present invention will be further described in detail below with reference to examples and drawings, but the embodiments of the present invention are not limited thereto.

Example 1

A method for testing the distribution of content and state of water in plant tissues by Raman spectroscopy is provided, comprising the following steps:

(1) Using a sampler to take a 12 mm × 15 mm (diameter × height) sample column from an apple in the radial direction, then using a self-made slicing device to cut the sample column into slices of 2 mm in thickness, then placing the slices immediately in a quartz chamber with constant temperature and humidity, and then sealing the chamber with a quartz cover glass of 0.3 mm in thickness; setting the humidity of the chamber to 80% and the temperature to 4 °C, and then placing the chamber on the stage of a laser confocal Raman microscope for testing.

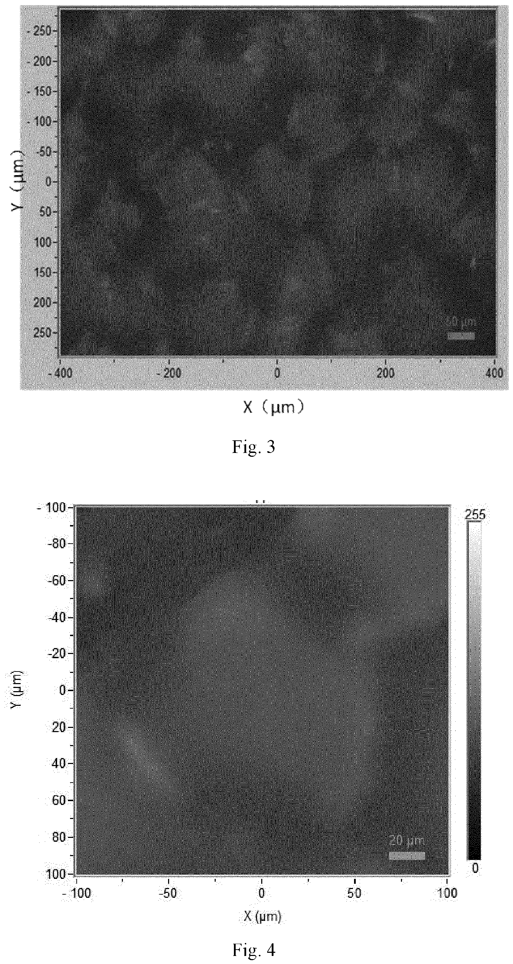

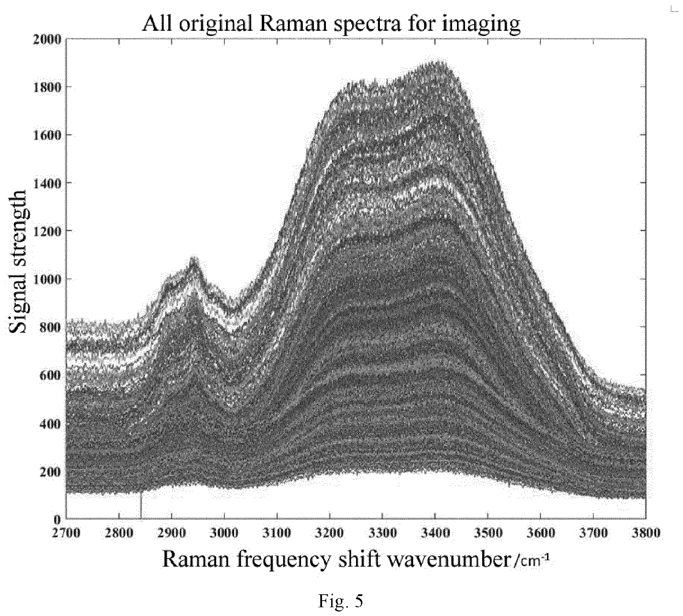

(2) Aligning the 10× objective lens of the laser confocal Raman microscope to observe the sample, and adjusting the focal length to obtain a clear microscopic image of the tissue structure; selecting cells with a complete structure as the measurement regions (200 $\mu$m × 200 $\mu$m in size, as shown in Fig. 4), and then dividing the selected regions into a grid with a step size of 5 $\mu$m to obtain a total of 1681 intersection points. The light source of the laser confocal Raman microscope, as a point light source, scanned from top to bottom and from left to right along the intersection points of the grid to acquire the Raman spectrum generated at each intersection point, so each spectrum had the corresponding coordinate information in the measurement region; for the spectrum acquisition, the spectral range was set to 2700-3800 $cm^{-1}$, a 532 nm laser was used, the size of the grid was 600 gr/mm, the Hole was 500, the laser energy attenuation was 25%, the acquisition time was 3 s, the accumulation times were three times, and the acquisition depth was 50 $\mu$m; the time required to complete all the scanning was about 3 h. All the original Raman spectra acquired were shown in Fig. 5.

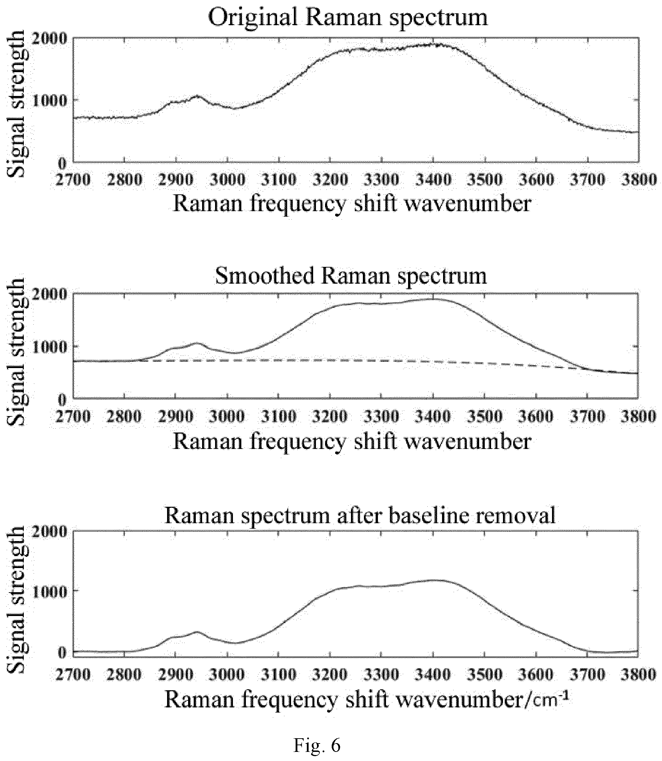

(3) All the acquired original Raman spectra were smoothed by the Savitzky-golay smoothing method and removed the baseline by the airPLS method; the Savitzky-golay Smooth function in the Matlab software was used for smoothing, wherein the window size of the Savitzky-golay algorithm used to smooth the spectrum was set to 15, and the degree of polynomial in the Savitzky-golay algorithm was 1; airPLS (adaptive iteratively reweighted penalized least squares), used to remove the baseline of the spectrum, which could well remove the baseline of the spectrum and retain the effective information of the spectrum to the maximum extent; in the airPLS algorithm, the parameter $\lambda$ was set as $10e^{12}$, the order of the differential penalized term was 3, the weight anomaly ratio at the start and end of the spectrum was set to 0.08, the asymmetry parameter at the start and end was 50, and the maximum number of iteration times was set to 10. Fig. 6 shows the spectrum of a certain original Raman spectrum after being preprocessed by smoothing and removing the baseline.

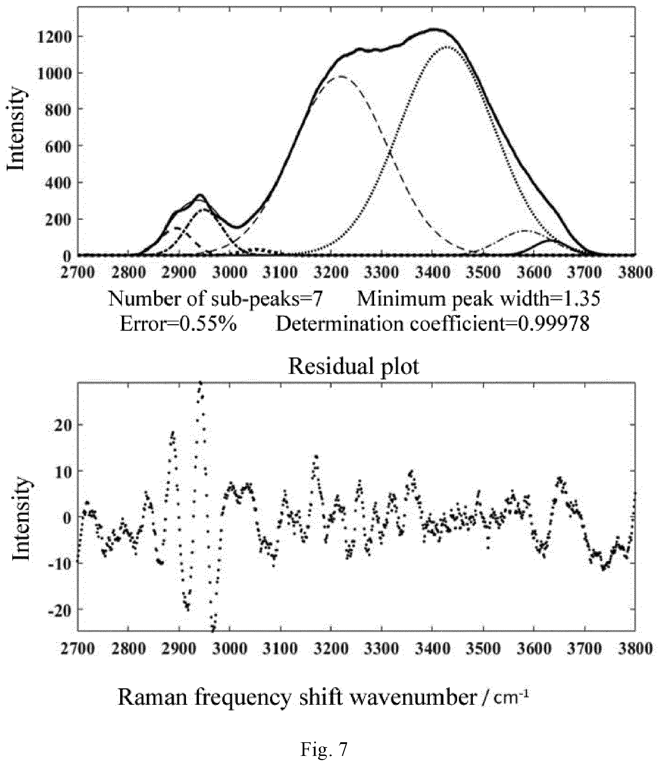

(4) Performing the Gaussian peak fitting on the preprocessed Raman spectra. Because the components of the sample was complex, it would have a certain impact on the spectrum, which made the peak positions of the spectrums different after the Gaussian peak fitting, not conducive to the subsequent data processing; therefore, the method of fixed-peak-position peak-separation fitting was used to fit all the spectra. The method to determine the peak positions was as follows: Randomly selecting fifty preprocessed Raman spectra, then using the software Peakfit v4.12 (Seasolve software Inc.) to decompose the spectrum into several sub-peaks, and obtaining 7 sub-peaks in the spectral range of 2700-3800 $cm^{-1}$ according to the peak fitting determination coefficient and the number of iterations, wherein five sub-peaks in the spectral range of 3000-3800 $cm^{-1}$ were the OH stretching vibration peaks; averaging the peak positions of the 7 sub-peaks of the fifty preselected spectra to obtain the peak position information of the fixed-peak-position peak fitting; performing peak fitting on all the preprocessed spectra through the Matlab software with the Peakfit function according to the obtained peak position information, so as to obtain the peak position, peak height, peak width and peak area of the sub-peaks of all the spectra after the peak separation, as well as the determination coefficient of the fixed-peak-position peak fitting of each spectrum, the relative error between the fitted spectrum and the original spectrum, the fitting residual error, and other information. The peak fitting results of a certain Raman spectrum are shown in Fig. 7 and Table 1.

Table 1. The peak position, peak height, peak width and peak area of the sub-peaks of a certain Raman spectrum after peak separation

| Sub-peak No. | Peak position | Peak height | Peak width | Peak area |
|---|---|---|---|---|
| 1 | 2895 | 148.9 | 73.2 | 11636.29 |
| 2 | 2950 | 249.8 | 83.7 | 22228.41 |
| 3 | 3053 | 30.61 | 69.17 | 2178.08 |
| 4 | 3219 | 978 | 218.8 | 227970.64 |
| 5 | 3428 | 1159 | 223 | 270103.66 |
| 6 | 3583 | 134.4 | 121.4 | 17502.96 |
| 7 | 3633 | 81.28 | 76.72 | 6648.71 |

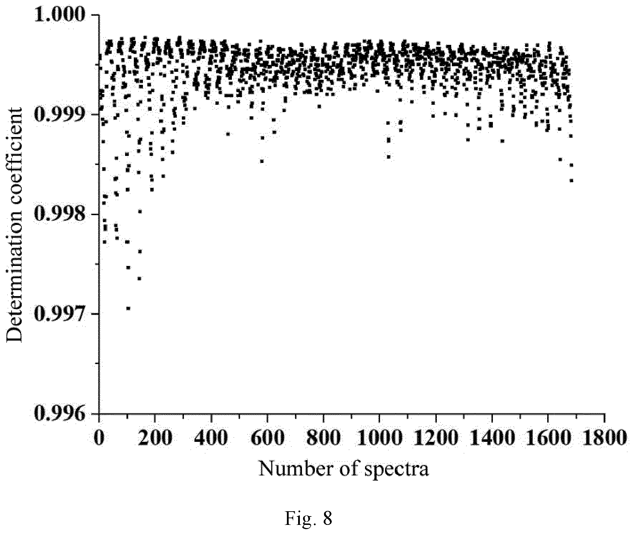

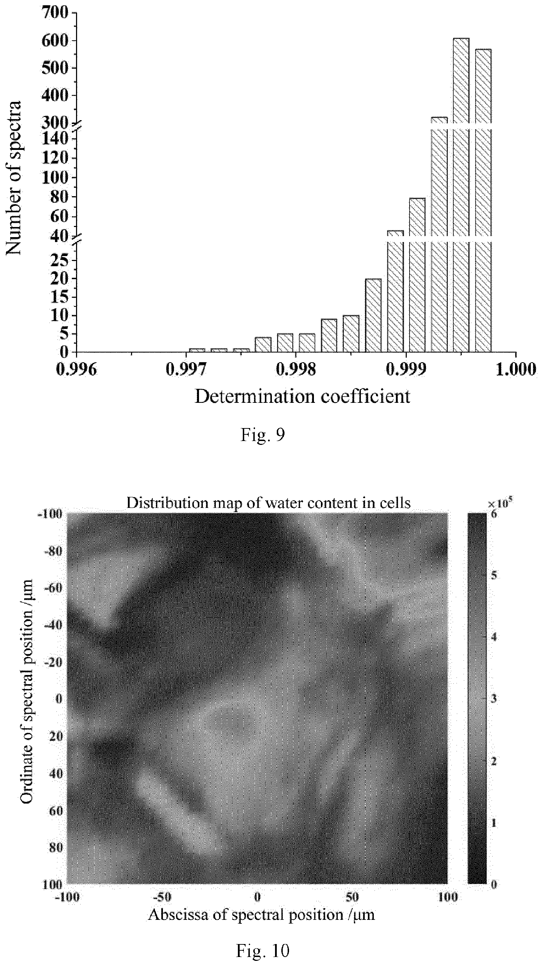

(5) Summing up the peak areas of the five sub-peaks at 3000-3800 $cm^{-1}$ after the peak fitting to obtain the sum A of the peak areas of each Raman spectrum at 3000-3800 $cm^{-1}$, then the A was the content of water molecules. In the five sub-peaks at 3000-3800 $cm^{-1}$, the ratio R of the peak area of the third sub-peak (centered at 3428 $cm^{-1}$) to the peak area of the second sub-peak (centered at 3219 $cm^{-1}$) could be used to determine the bonding state of water molecules; the number of the peak-fitting spectra of all the spectra was 1681. The fitting determination coefficient obtained was shown in Fig. 8, and the histogram of the distribution of the determination coefficient was shown in Fig. 9.

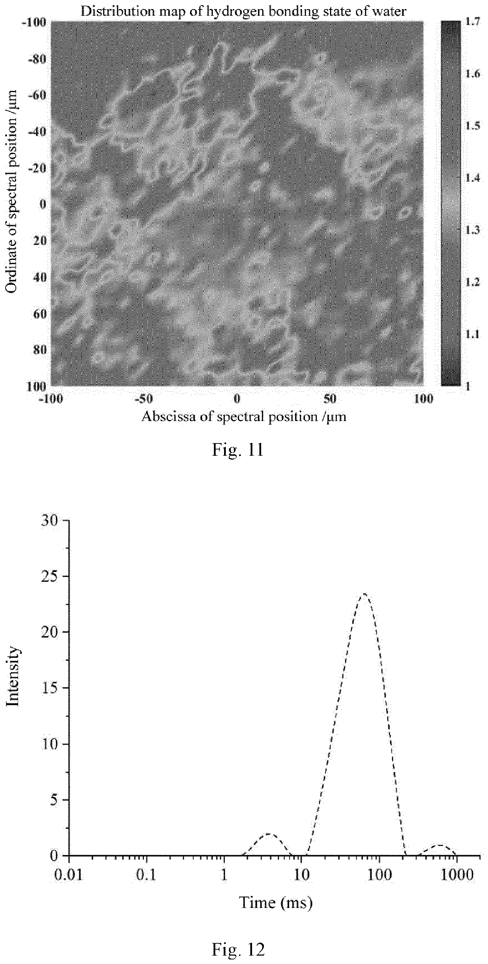

(6) combining the content of water molecules A and the corresponding coordinate information to perform pseudocolor imaging by using the Matlab software as shown in Fig. 10, to obtain the water distribution map at the cellular level in the selected range. The larger the value of A was, the higher the water content at this point was; while the smaller the value of A was, the lower the water content at this point was. Besides, the ratio R could be used as the pixel to judge the hydrogen bonding state of water at each point, as shown in Fig. 11. The larger the value of R was, the lower the bonding degree was; while the smaller the value of R was, the higher the binding degree was. Thus, the visualization of the distribution of water content and water binding state at the cellular level in fruit and vegetable tissues could be realized. The Matlab pseudocolor imaging used its own Pcolor and Colormap functions; and Shading interp was used for shading.

According to the value of R of water in Fig. 11, water with R value smaller than 1.2 was defined as bound water, water with R value greater than 1.4 was defined as free water, and water with R value between 1.2 and 1.4 was defined as immobilized water. The content of water with different states was calculated based on the corresponding coordinate information according to the water content in Fig. 10. Taking three cylinders in each apple and then three slices in each cylinder, then testing all the slices by the method of this application to obtain 9 groups of data, and then analyzing and calculating these data; averaging the values of free water, bound water and immobilized water, respectively, and comparing these average values with the results measured by the NMR method (measuring three identical cylinders on the same apple and averaging the values, i.e., the test sample was the same as the sample used in the method of this application) and the Marlin Chick experiment (two groups of the same apple were tested, and three identical cylinders were obtained from each group, i.e., the test samples were the same as the samples used in the method of this application, one group used for the sucrose dipping test, the other used for the experiment of water measurement by drying, and then the obtained results were averaged), respectively. The results were shown in Table 1.

The NMR method was as follows:

① Taking three sample columns with a size of 12 mm × 15 mm (diameter × height) from different parts of the same apple along the radial direction, then placing the sample columns in an NMR test tube with a diameter of 20 mm, and then sealing the tube with a parafilm and placing in a refrigerator to stay at a constant temperature of 4°C for 2 h.

② A low-field NMR instrument was used in this experiment; before the test, a standard sample was used to calibrate the instrument, and an FID sequence was used to find the center frequency, then a CPMG test sequence was selected, and the testing parameters are as follows: SW = 200 kHz, RFD = 0.02 μs, RG1 = 5, DRG1 = 3, DR = 1, PRG = 0, NS = 3, TW = 8000 ms, TE = 0.5 ms, NECH = 2500.

③ Putting the sample into a magnet box, and selecting the cumulative sampling to collect the $T_2$ relaxation signal of the sample; after the sampling, the data would be automatically saved in the database; selecting the measured data to perform data inversion to obtain the final results. In the inversion results, the peak of the $T_2$ relaxation peak in the range of 100-1000 ms was considered as the peak of free water in the sample, and the corresponding percentage of the peak area was the percentage of free water; the peak of the $T_2$ relaxation peak in the range of 10-100 ms was considered as the peak of immobilized water, and the corresponding percentage of the peak area was the percentage of immobilized water; the peak of the $T_2$ relaxation peak in the range of 0-10 ms was considered as the peak of bound water, and the corresponding percentage of the peak area was the percentage of bound water. The results were shown in Fig. 12.

The Marlin Chick experiment was performed as follows:

① Taking six sample columns with a size of 12 mm × 15 mm (diameter × height) from different parts of the same apple along the radial direction; cutting three of the sample columns into sample discs with a thickness of 2 mm, then respectively placing the sample discs in three weighing dishes with a known mass of $m_0$, then respectively weighing the total mass $m_1$ of the three weighing dishes, then placing the three weighing dishes in an oven to dry at 105°C for 10 h to a constant weight, and then weighing the total mass $m_2$; calculating the water content of the

$$\text{water content of tissue (\%)} = \frac{(m_1 - m_2)}{(m_1 - m_0)} \times 100$$

tissue according to the following formula:

② Also cutting the other three sample columns into sample discs with a thickness of 2 mm, and respectively placing the sample discs in the other three weighing dishes with the known mass of $M_0$ to obtain the total mass $M_1$; using a pipette to add a sucrose solution with a mass percent concentration of 60% respectively into the three weighing dishes, then gently shaking the weighing dish to make the solution and sample mixed uniformly, and then weighing its mass $M_2$.

③ Placing the weighing dish on a rotary oscillator to oscillate for 6 h, and setting the rotation rate of the oscillator so that the solution in the weighing dish could be gently shaken in one direction without spilling out of the weighing dish.

④ After the oscillation, fully shaking the solution, then using a pipette to drop 200 μL of the sample on the ground glass surface of the Abbe refractometer, and then screwing the prism tightly; measuring the sugar concentration $D_2$ of the solution at 20°C, then measuring the original sugar concentration $D_1$, and then calculating the free water content (%) in the tissue according to the following formula: free water

$$\text{content of tissue(\%)} = \frac{(M_2 - M_1) \times (D_1 - D_2)}{(M_1 - M_0) \times D_2} \times 100$$

. Thus, the percentage (%) of the bound water content of the total water content = (water content of tissue - free water content of tissue) × 100/water content of tissue, this percentage is the bound water content; and the proportion of free water in total water content = 1 - bound water content of the total water.

(7) Table 2 shows the comparison between the contents of bound water and free water measured by the method of this application and the contents of bound water and free water measured by the Marlin Chick experiment and NMR.

## Table 2. The contents of bound water and free water in an apple measured by different test methods

| Water state    Test method | Free water | Immobilized water | Bound water |
|---|---|---|---|
| The method of this application | $8.8 \pm 2.1\%$ | $83.1 \pm 4.7\%$ | $8.1 \pm 1.8\%$ |
| NMR method | $8.5 \pm 1.7\%$ | $85.6 \pm 6.2\%$ | $5.9 \pm 1.3\%$ |
| Marlin Chick method | $89.9 \pm 7.2\%$ | | $10.1 \pm 5.1\%$ |

The above examples are preferred embodiments of the present invention, but the embodiments of the present invention are not limited thereto, and any other alterations, modifications, replacements, combinations and simplifications should be equivalent substitutions and included in the scope of protection of the present invention The invention is only limited by the appended claims.

**Claims**

1.  A method for testing cellular level water content and distribution in fruit and vegetable tissues based on Raman spectroscopy, **characterized in that** the method comprises the following steps:

    (1) cutting fruits and vegetables for testing into a sample;
    (2) placing the sample in a stage of a laser confocal microscope for imaging spectrum acquisition; specifically, selecting a cell region from the sample by an objective lens of the laser confocal microscope, then meshing the selected cell region to obtain uniformly distributed intersection points, then marking the corresponding coordinate information of each intersection point in the selected cell region, and then scanning each intersection point to obtain the corresponding Raman spectrum of water at each intersection point in the cell region; wherein the step size of the grid is 3-5 $\mu$m;
    (3) processing the Raman spectra obtained in step (2) for smoothing noise reduction and removing fluorescence background, and then performing the Gaussian peak fitting; wherein five sub-peaks at 3000-3800 cm$^{-1}$ and two or three sub-peaks at 2700-3000 cm$^{-1}$ are obtained for each Raman spectrum;
    (4) summing up the areas of the five sub-peaks at 3000-3800 cm$^{-1}$ after the peak fitting of each Raman spectrum to obtain the corresponding water content A at the intersection point in the cell region, and then determining the bonding state of the corresponding water molecules at the intersection point according to the ratio R of the peak area of the sub-peak centered at 3410-3440 cm$^{-1}$ to the peak area of the sub-peak centered at 3200-3220 cm$^{-1}$; and
    (5) by combining with the coordinate information of each intersection point, using the corresponding water content A and ratio R at all the intersection points as pixels for pseudocolor imaging to obtain the distribution of water content and water bonding state at the cell level in the fruit and vegetable tissues.

2.  The method for testing cellular level water content and distribution in fruit and vegetable tissues based on Raman spectroscopy according to claim 1, **characterized in that**: the laser used for the imaging spectrum acquisition in step (2) is a 532 nm laser, wherein the size of the grid is 1200 gr/mm, the Hole is 500, and the scanning range is 2700-3800 cm$^{-1}$; the acquisition conditions are as follows: the acquisition time is 3-5 s, the accumulation times are 2-3 times, and the laser energy attenuation is 25% to 50%.

3.  The method for testing cellular level water content and distribution in fruit and vegetable tissues based on Raman spectroscopy according to claim 1, **characterized in that**: the imaging spectrum acquisition in step (2) is performed at a depth of 50-100 $\mu$m.

4.  The method for testing cellular level water content and distribution in fruit and vegetable tissues based on Raman spectroscopy according to claim 1, 2 or 3, **characterized in that**: the "Gaussian peak fitting" in step (3) is carried out by using the Matlab software with the Peakfit function.

5.  The method for testing cellular level water content and distribution in fruit and vegetable tissues based on Raman spectroscopy according to claim 4, **characterized in that**: in step (3), the "smoothing noise reduction" adopts the Savitzky-golay convolution smoothing algorithm in the Matlab software; and the "removing fluorescence background" adopts an adaptive iteratively reweighted penalized least squares background subtraction algorithm.

6.  The method for testing cellular level water content and distribution in fruit and vegetable tissues based on Raman spectroscopy according to claim 4, **characterized in that**: the "Gaussian peak fitting" in step (3) is a fixed-peak-position Gaussian curve fitting, that is, the Gaussian iterative curve fitting algorithm is used to perform the Gaussian curve fitting on the spectrum in the case of fixed peak position; the method for determining the peak position is as follows: randomly selecting fifty Raman spectra, and using the Peakfit software to perform iterative peak fitting to decompose each Raman spectrum into seven or eight sub-peaks; then averaging the peak position information of the sub-peaks obtained from the fifty Raman spectra to get the average peak position information of the seven or eight sub-peaks, which can be used as the peak-position of the fixed-peak-position Gaussian peak separation.

7.  The method for testing cellular level water content and distribution in fruit and vegetable tissues based on Raman spectroscopy according to claim 4, **characterized in that**: the size of the cell region in step (2) is determined by the size of the cells, and the average diameter of the conventional fruit and vegetable cells is 100-300 $\mu$m; the magnification of the objective lens is 10$\times$.

8.  The method for testing cellular level water content and distribution in fruit and vegetable tissues based on Raman spectroscopy according to claim 4, **characterized in that**: the fruits and vegetables in step (1) are one of apples, potatoes, grapes, pears and cabbage stems.

9. The method for testing cellular level water content and distribution in fruit and vegetable tissues based on Raman spectroscopy according to claim 8, **characterized in that**: the sample in step (1) is peeled fruits and vegetables; the shape of the sample is of a disc, having a size of diameter $\times$ thickness = 12 mm $\times$ 2 mm; before and during the test, the sample is stored in a quartz chamber that, sealed with a quartz cover glass of 0.3 mm in thickness, has a temperature of 2 °C to 10°C and a humidity greater than 80%.

10. The method for testing cellular level water content and distribution in fruit and vegetable tissues based on Raman spectroscopy according to claim 4, **characterized in that**: the "pseudocolor imaging" in step (5) is carried out by using the Matlab software with the Pcolor and Colormap functions, wherein Shading interp is used for shading.

**Patentansprüche**

1. Ein Verfahren zum Testen des Wassergehalts und der Wasserverteilung auf zellulärer Ebene in Obst- und Gemüsegeweben, basierend auf Raman-Spektroskopie, **dadurch gekennzeichnet, dass** das Verfahren die folgenden Schritte umfasst

    (1) Schneiden von Obst und Gemüse zum Testen in eine Probe;
    (2) Einsetzen der Probe in eine Bühne eines Laserkonfokalmikroskops zur bildgebenden Spektrumerfassung; insbesondere Auswählen eines Zellbereichs aus der Probe durch eine Objektivlinse des Laserkonfokalmikroskops, dann Vermaschung des ausgewählten Zellbereichs, um gleichmäßig verteilte Schnittpunkte zu erhalten, dann Markieren der entsprechenden Koordinateninformation jedes Schnittpunkts in dem ausgewählten Zellbereich, und dann Scannen jedes Schnittpunkts, um das entsprechende Raman-Spektrum von Wasser an jedem Schnittpunkt in dem Zellbereich zu erhalten;
    wobei die Schrittgröße des Rasters 3-5 $\mu$m ist;
    (3) Verarbeiten der in Schritt (2) erhaltenen Raman-Spektren zum Glätten der Rauschreduktion und Entfernen des Fluoreszenzhintergrunds und anschließendes Durchführen der Gauß'schen Peak-Anpassung, wobei für jedes Raman-Spektrum fünf Teilpeaks bei 3000-3800 cm$^{-1}$ und zwei oder drei Teilpeaks bei 2700-3000 cm$^{-1}$ erhalten werden;
    (4) Aufsummieren der Flächen der fünf Teilpeaks bei 3000-3800 cm$^{-1}$ nach der Peak-Anpassung jedes Raman-Spektrums, um den entsprechenden Wassergehalt A am Schnittpunkt in der Zellenregion zu erhalten, und dann Bestimmen des Bindungszustands der entsprechenden Wassermoleküle am Schnittpunkt gemäß dem Verhältnis R der Peakfläche des bei 3410-3440 cm$^{-1}$ zentrierten Teilpeaks zur Peakfläche des bei 3200-3220 cm$^{-1}$ zentrierten Teilpeaks; und
    (5) durch Kombinieren mit den Koordinateninformationen jedes Schnittpunkts, unter Verwendung des entsprechenden Wassergehalts A und des Verhältnisses R an allen Schnittpunkten als Pixel für die Pseudofarbbildgebung, um die Verteilung des Wassergehalts und des Wasserbindungszustands auf der Zellebene in den Obst- und Gemüsegeweben zu erhalten.

2. Das Verfahren zum Testen des Wassergehalts und der Wasserverteilung auf zellulärer Ebene in Obst- und Gemüsegeweben basierend auf Raman-Spektroskopie gemäß Anspruch 1, **dadurch gekennzeichnet, dass**: der Laser, der für die bildgebende Spektrumerfassung in Schritt (2) verwendet wird, ein 532-nm-Laser ist, wobei die Größe des Rasters 1200 grimm, das Loch 500 und der Scan-Bereich 2700-3800 cm$^{-1}$ ist; die Erfassungsbedingungen wie folgt sind: die Erfassungszeit ist 3-5 s, die Akkumulationszeiten sind 2-3 mal, und die Laserenergieabschwächung ist 25% bis 50%.

3. Das Verfahren zum Testen des Wassergehalts und der Wasserverteilung auf zellulärer Ebene in Obst- und Gemüsegeweben basierend auf Raman-Spektroskopie gemäß Anspruch 1, **dadurch gekennzeichnet, dass**: die bildgebende Spektrumerfassung in Schritt (2) in einer Tiefe von 50-100 $\mu$m durchgeführt wird.

4. Das Verfahren zum Testen des Wassergehalts und der Wasserverteilung auf zellulärer Ebene in Obst- und Gemüsegeweben basierend auf Raman-Spektroskopie gemäß Anspruch 1, 2 oder 3, **dadurch gekennzeichnet, dass**: die "Gauß'sche Peak-Anpassung" in Schritt (3) unter Verwendung der Matlab-Software mit der Peakfit-Funktion durchgeführt wird,

**5.** Das Verfahren zum Testen des Wassergehalts und der Wasserverteilung auf zellulärer Ebene in Obst- und Gemüsegeweben basierend auf Raman-Spektroskopie gemäß Anspruch 4, **dadurch gekennzeichnet, dass** in Schritt (3) die "Glättungsrauschreduktion" den Savitzky-Golay-Faltungsglättungsalgorithmus in der Matlab-Software anwendet; und die "Entfernung des Fluoreszenzhintergrunds" einen adaptiven, iterativ neu gewichteten, bestraften Kleinstquadrat-Hintergrundsubtraktionsalgorithmus anwendet.

**6.** Das Verfahren zum Testen des Wassergehalts und der Wasserverteilung auf zellulärer Ebene in Obst- und Gemüsegeweben basierend auf Raman-Spektroskopie gemäß Anspruch 4, **dadurch gekennzeichnet, dass**: die "Gauß'sche Peak-Anpassung" in Schritt (3) eine Gauß'sche Kurvenanpassung mit fester Peak-Position ist, d.h. der Gauß'sche iterative Kurvenanpassungsalgorithmus wird verwendet, um die Gauß'sche Kurvenanpassung an dem Spektrum im Fall einer festen Peak-Position durchzuführen; das Verfahren zum Bestimmen der Peak-Position ist wie folgt: Auswahl von fünfzig Raman-Spektren nach dem Zufallsprinzip und Verwendung der Peakfit-Software zur Durchführung der iterativen Peak-Anpassung, um jedes Raman-Spektrum in sieben oder acht Teilpeaks zu zerlegen; anschließend Mittelung der Peak-Positionsinformationen der aus den fünfzig Raman-Spektren erhaltenen Teilpeaks, um die durchschnittlichen Peak-Positionsinformationen der sieben oder acht Teilpeaks zu erhalten, die als Peak-Position der Gaußschen Peak-Trennung mit fester Peak-Position verwendet werden können.

**7.** Ein Verfahren zur Untersuchung des Wassergehalts und der Wasserverteilung auf zellulärer Ebene in Obst- und Gemüsegeweben basierend auf Raman-Spektroskopie gemäß Anspruch 4, **dadurch gekennzeichnet, dass**: die Größe des Zellbereichs in Schritt (2) durch die Größe der Zellen bestimmt wird und der durchschnittliche Durchmesser der herkömmlichen Obst- und Gemüsezellen 100-300 μm beträgt; die Vergrößerung der Objektivlinse 10 × beträgt.

**8.** Das Verfahren zum Testen des Wassergehalts und der Wasserverteilung auf zellulärer Ebene in Obst- und Gemüsegeweben gemäß Anspruch 4, **dadurch gekennzeichnet, dass**: die Früchte und Gemüse in Schritt (1) eines von Äpfeln, Kartoffeln, Trauben, Birnen und Kohlsorten sind.

**9.** Das Verfahren zum Testen des Wassergehalts und der Wasserverteilung auf zellulärer Ebene in Obst- und Gemüsegeweben basierend auf Raman-Spektroskopie gemäß Anspruch 8, **dadurch gekennzeichnet, dass**: die Probe in Schritt (1) aus geschältem Obst und Gemüse besteht; die Form der Probe eine Scheibe mit einer Größe von Durchmesser × Dicke = 12 mm × 2 mm ist; die Probe vor und während des Tests in einer Quarzkammer aufbewahrt wird, die mit einem 0,3 mm dicken Quarzglas verschlossen ist und eine Temperatur von 2°C bis 10°C und eine Luftfeuchtigkeit von mehr als 80% aufweist.

**10.** Das Verfahren zum Testen des Wassergehalts und der Wasserverteilung auf zellulärer Ebene in Obst- und Gemüsegeweben basierend auf Raman-Spektroskopie gemäß Anspruch 4, **dadurch gekennzeichnet, dass**: die "Pseudofarbbildgebung" in Schritt (5) unter Verwendung der Matlab-Software mit den Funktionen Pcolor und Colormap durchgeführt wird, wobei Shading interp zur Schattierung verwendet wird.

**Revendications**

**1.** Procédé de test de la teneur et distribution d'eau au niveau cellulaire dans des tissus de fruit et légume basé sur la spectroscopie Raman, **caractérisé en ce que** le procédé comprend les étapes suivantes :

(1) découper des fruits et légumes pour le test en un échantillon ;
(2) placer l'échantillon dans une platine d'un microscope confocal laser pour une acquisition de spectre d'imagerie ; spécifiquement, sélectionner une région cellulaire dans l'échantillon par une lentille de focalisation du microscope confocal laser, puis mailler la région cellulaire sélectionnée pour obtenir des points d'intersection à distribution uniforme, puis marquer les informations de coordonnées correspondantes de chaque point d'intersection dans la région cellulaire sélectionnée, puis balayer chaque point d'interaction pour obtenir le spectre Raman correspondant de l'eau à chaque point d'intersection dans la région cellulaire ;
dans lequel la taille de pas de la grille est de 3 à 5 μm ;
(3) traiter les spectres Raman obtenus à l'étape (2) pour lisser la réduction de bruit et éliminer le fond de fluorescence, puis réaliser l'ajustement de pic gaussien ; dans lequel cinq sous-pics à 3000 à 3800 cm$^{-1}$ et deux ou trois sous-pics à 2700 à 3000 cm$^{-1}$ sont obtenus pour chaque spectre Raman ;
(4) additionner les aires des cinq sous-pics à 3000 à 3800 cm$^{-1}$ après l'ajustement de pic de chaque spectre Raman pour obtenir la teneur en eau correspondante A au point d'intersection dans la région cellulaire, puis déterminer l'état de liaison des molécules d'eau correspondantes au point d'intersection en fonction du rapport R de l'aire de pic du sous-pic centré à 3410 à 3440 cm$^{-1}$ sur l'aire de pic du sous-pic centré à 3200 à 3220 cm$^{-1}$ ; et

(5) en combinant avec les informations de coordonnées de chaque point d'intersection, utiliser la teneur en eau A et le rapport R correspondants à l'ensemble des points d'intersection en tant que pixels pour l'imagerie en pseudo-couleur pour obtenir la distribution de teneur en eau et l'état de liaison d'eau au niveau cellulaire dans les tissus de fruit et légume.

2. Procédé de test de la teneur et distribution d'eau au niveau cellulaire dans des tissus de fruit et légume basé sur la spectroscopie Raman selon la revendication 1, **caractérisé en ce que** : le laser utilisé pour l'acquisition de spectre d'imagerie à l'étape (2) est un laser de 532 nm, dans lequel la taille de la grille est de 1200 gr/mm, le trou est de 500, et la gamme de balayage est de 2700 à 3800 cm$^{-1}$ ; les conditions d'acquisition sont comme suit : le temps d'acquisition est de 3 à 5 s, les temps d'accumulations sont de 2 à 3 fois, et l'atténuation d'énergie laser est de 25 % à 50 %.

3. Procédé de test de la teneur et distribution d'eau au niveau cellulaire dans des tissus de fruit et légume basé sur la spectroscopie Raman selon la revendication 1, **caractérisé en ce que** : l'acquisition de spectre d'imagerie à l'étape (2) est réalisée à une profondeur de 50 à 100 $\mu$m.

4. Procédé de test de la teneur et distribution d'eau au niveau cellulaire dans des tissus de fruit et légume basé sur la spectroscopie Raman selon la revendication 1, 2 ou 3, **caractérisé en ce que** : « l'ajustement de pic gaussien » à l'étape (3) est réalisé en utilisant le logiciel Matlab avec la fonction Peakfit.

5. Procédé de test de la teneur et distribution d'eau au niveau cellulaire dans des tissus de fruit et légume basé sur la spectroscopie Raman selon la revendication 4, **caractérisé en ce que** : à l'étape (3), le « lissage de réduction de bruit » adopte l'algorithme de lissage de convolution Savitzky-Golay dans le logiciel Matlab ; et « l'élimination du fond de fluorescence » adopte un algorithme de soustraction de fond des moindres carrés pénalisé, repondéré de manière itérative, adaptatif.

6. Procédé de test de la teneur et distribution d'eau au niveau cellulaire dans des tissus de fruit et légume basé sur la spectroscopie Raman selon la revendication 4, **caractérisé en ce que** : « l'ajustement de pic gaussien » à l'étape (3) est un ajustement de courbe gaussienne à position de pic fixe, c'est-à-dire que l'algorithme d'ajustement de courbe itératif gaussien est utilisé pour réaliser l'ajustement de courbe gaussienne sur le spectre en cas de position de pic fixe ; le procédé pour déterminer la position de pic est comme suit : sélectionner aléatoirement cinquante spectres Raman, et utiliser le logiciel Peakfit pour réaliser un ajustement de pic itératif pour décomposer chaque spectre Raman en sept ou huit sous-pics ; puis moyenner les informations de position de pic des sous-pics obtenues à partir des cinquante spectres Raman pour obtenir les informations de position de pic moyenne des sept ou huit sous-pics, qui peuvent être utilisées en tant que position de pic de la séparation de pic gaussien à position de pic fixe.

7. Procédé de test de la teneur et distribution d'eau au niveau cellulaire dans des tissus de fruit et légume basé sur la spectroscopie Raman selon la revendication 4, **caractérisé en ce que** : la taille de la région cellulaire à l'étape (2) est déterminée par la taille des cellules, et le diamètre moyen des cellules de fruit et légume conventionnelles est de 100 à 300 $\mu$m ; le grossissement de la lentille de focalisation est de 10x.

8. Procédé de test de la teneur et distribution d'eau au niveau cellulaire dans des tissus de fruit et légume basé sur la spectroscopie Raman selon la revendication 4, **caractérisé en ce que** : les fruits et légumes à l'étape (1) sont des fruits et légumes parmi des pommes, pommes de terre, raisins, poires et tiges de chou.

9. Procédé de test de la teneur et distribution d'eau au niveau cellulaire dans des tissus de fruit et légume basé sur la spectroscopie Raman selon la revendication 8, **caractérisé en ce que** : l'échantillon à l'étape (1) est des fruits et légumes épluchés ; la forme de l'échantillon est celle d'un disque ayant une taille de diamètre $\times$ épaisseur = 12 mm $\times$ 2 mm ; avant et au cours du test, l'échantillon est stocké dans une chambre de quartz qui, scellée avec un verre de recouvrement en quartz de 0,3 mm d'épaisseur, a une température de 2 °C à 10 °C et une humidité supérieure à 80 %.

10. Procédé de test de la teneur et distribution d'eau au niveau cellulaire dans des tissus de fruit et légume basé sur la spectroscopie Raman selon la revendication 4, **caractérisé en ce que** : « l'imagerie en pseudo-couleur » à l'étape (5) est réalisée en utilisant le logiciel Matlab avec les fonctions Pcolor et Colormap, dans lequel Shading interp est utilisée pour l'ombrage.

Fig. 1

Fig. 2

Fig. 3

Fig. 4

All original Raman spectra for imaging

Fig. 5

Fig. 6

Number of sub-peaks=7     Minimum peak width=1.35
Error=0.55%     Determination coefficient=0.99978

Residual plot

Raman frequency shift wavenumber / cm⁻¹

Fig. 7

Fig. 8

Fig. 9

Fig. 10

Distribution map of hydrogen bonding state of water

Fig. 11

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3164046 A1

**Non-patent literature cited in the description**

- Vis-NIR hyperspectral imaging in visualizing moisture distribution of mango slices during microwave-vacuum dr. **PU YUAN-YUAN et al.** Food chemistry. Elsevier, 27 April 2015, vol. 188, 271-278

- **RAOUL VYUMVUHORE et al.** Effects of atmospheric relative humidity on Stratum Corneum structure at the molecular level: ex vivo Raman spectroscopy analysis. *Analyst,* 01 January 2013, vol. 138 (14), 4103